# EUROPEAN PATENT SPECIFICATION

(11) **EP 3 982 963 B1**
(45) Date of publication and mention of the grant of the patent: **12.11.2025**
(21) Application number: 20823347.8
(22) Date of filing: 12.06.2020
(51) Int. Cl.: A61K 31/451, A61K 31/44, A61K 31/4418, A61P 25/00, C07D 211/42, C07D 211/52, C07D 211/30, C07D 211/18, C07D 211/20, C07D 211/24, C07D 211/70, C07D 295/08, C07D 295/04, C07D 405/02, A61K 31/452

(54) **COMPOSITION COMPRISING PRIDOPIDINE AND ANALOG THEREOF FOR TREATING HUNTINGTON DISEASE AND SYMPTOMS THEREOF**
PRIDOPIDIN ENTHALTENDE ZUSAMMENSETZUNG UND ANALOGON DAVON ZUR BEHANDLUNG VON MORBUS HUNTINGTON UND SEINEN SYMPTOMEN
COMPOSITION COMPRENANT DE LA PRIDOPIDINE ET UN ANALOGUE DE CELLE-CI POUR TRAITER LA MALADIE DE HUNTINGTON ET SES SYMPTÔMES

(30) Priority: 12.06.2019 US 201916438508
(43) Date of publication of application: 20.04.2022
(73) Proprietor: Prilenia Neurotherapeutics Ltd., 6097200 Yakum (IL)
(72) Inventor: GEVA, Michal, Even-Yehuda 0000 (IL); BERELOVICH, Elena, Hadera 3843016 (IL); HAYDEN, Michael, Herzliya 4672561 (IL)
(74) Representative: Pearl Cohen Zedek Latzer Baratz UK LLP
(86) International application number: PCT/IL2020/050654
(87) International publication number: WO 2020/250234

(56) References cited:
- WO-A1-2018/039477
- US-A1- 2015 374 677
- US-A1- 2019 030 016
- REILMANN RALF ET AL: "Safety and efficacy of pridopidine in patients with Huntington's disease (PRIDE-HD): a phase 2, randomised, placebo-controlled, multicentre, dose-ranging study", THE LANCET NEUROLOGY, vol. 18, no. 2, 15 December 2018 (2018-12-15), AMSTERDAM, NL, pages 165 - 176, XP093046457, ISSN: 1474-4422, DOI: 10.1016/S1474-4422(18)30391-0
- REILMANN RALF ET AL: "Safety and efficacy of pridopidine in patients with Huntington's disease (PRIDE-HD): a phase 2, randomised, placebo-controlled, multicentre, dose-ranging study - Supplementary appendix", THE LANCET NEUROLOGY, vol. 18, no. 2, 15 December 2018 (2018-12-15), AMSTERDAM, NL, XP093046466, ISSN: 1474-4422, DOI: 10.1016/S1474-4422(18)30391-0
- COPPEN EMMA M. ET AL: "Current Pharmacological Approaches to Reduce Chorea in Huntington's Disease", DRUGS, vol. 77, no. 1, 17 December 2016 (2016-12-17), NZ, pages 29 - 46, XP093046345, ISSN: 0012-6667, DOI: 10.1007/s40265-016-0670-4
- BECHTEL N., SCAHILL R. I., ROSAS H. D., ACHARYA T., VAN DEN BOGAARD S. J. A., JAUFFRET C., SAY M. J., STURROCK A., JOHNSON H., ONO: "Tapping linked to function and structure in premanifest and symptomatic Huntington disease", NEUROLOGY, LIPPINCOTT WILLIAMS & WILKINS , PHILADELPHIA, US, vol. 75, no. 24, 14 December 2010 (2010-12-14), US, pages 2150 - 2160, XP055773601, ISSN: 0028-3878, DOI: 10.1212/WNL.0b013e3182020123
- ANON: "Unified Huntington’s Disease Rating Scale: Reliability and-Consis tenc y", MOVEMENT DISORDERS, MOVEMENT DISORDER SOCIETY, vol. 11, no. 2, 1 January 1996 (1996-01-01), pages 136 - 142, XP055677965

## Description

### FIELD OF THE INVENTION

This invention is directed to an oral pharmaceutical composition comprising pridopidine or a pharmaceutically acceptable salt thereof and an analog thereof or a pharmaceutically acceptable salt thereof for use in treating Huntington disease.

### BACKGROUND OF THE INVENTION

### Huntington disease

Huntington disease (HD) is a fatal neurodegenerative disorder with an autosomal dominant mode of inheritance. The disease is associated with motor, behavioral, functional and cognitive symptoms. Motor disturbances are the defining feature of the disease, with chorea the most evident motor symptom. Although useful for diagnosis, chorea is a poor marker of disease severity. Rather, functional disability and disease severity best correlate with negative motor features such as impairment in fine motor skills, bradykinesia, and gross motor coordination skills, including speech difficulties, gait, and postural dysfunction as well as cognitive impairments (1). The UHDRS-Total Functional Capacity (TFC) scale is a validated and accepted tool used by clinicians to assess HD disease stage and the level of patient's functionality. A decline in UHDRS-TFC is associated with other aspects of disease progression including brain atrophy, motor, cognitive and behavioural functions (2,3).

The HD includes five stages:
**HD1** (TFC 11-13), 0-8 years after onset. Early stage. Fully functional at home and at work, maintains typical pre-disease levels of independence when it comes to everyday activities.
**HD2** (TFC 7-10), 3-13 years after onset. Early intermediate stage. Patient is still functional at work, but at a lower capacity.
**HD3** (TFC 3-6) 5-16 years after onset. Late intermediate stage. Patient can no longer conduct work or manage household responsibilities.
**HD4** (TFC 1-2) 9-21 years after onset. Early advanced stage. Patient is not independent at this stage, but still can reside in their home with help from either family or professionals, although their needs may be better met at an extended care facility.
**HD5** (TFC 0) 11-26 years after onset, advanced stage. Patient needs total support in daily activities from professional nursing care.

A number of medications are prescribed to ameliorate the motor and emotional problems associated with HD. However, there is little scientific evidence for the usefulness of various drugs in HD (4,5). Only two drugs, tetrabenazine and deutetrabenazine, are approved to treat chorea in HD patients, but no therapy has yet proved able to modify the progressive and inexorable functional decline of the disease (6,7). As such, there is a significant unmet medical need to develop medications to ameliorate symptoms of HD, and to delay disease progression.

### Pridopidine

Pridopidine (4-[3-(methylsulfonyl)phenyl]-1-propyl-piperidine) is a highly selective S1R ligand with Ki=0.57 nM and S2R Ki of 5450 nM(8). Thus, pridopidine has 95-fold higher affinity for the S1R vs the S2R and is a highly selective S1R ligand.

The S1R is an endoplasmic reticulum (ER) protein located mainly at the mitochondria-associated membrane (MAM), modulating diverse cellular processes including calcium signaling, ion-channel activity, and the ER stress response (9,10). Pridopidine demonstrates neuroprotective properties mediated by the S1R in several in vivo and in vitro HD models, including a robust and dose-dependent neuroprotective effect against mutant huntingtin-(mHTT)-induced cell death in human HD induced pluripotent stem cells (iPSCs) and mouse HD cortical neurons (11). In HD cortico-striatal cultures, pridopidine increases brain-derived neurotrophic factor (BDNF) transport, enhances synaptic activity, and promotes phosphorylation of the pro-survival protein extracellular signal-regulated kinase (ERK)(12,13). Pridopidine also enhances secretion of the neuroprotective brain-derived neurotrophic factor (BDNF) in a neuroblastoma cell line, in a S1R-dependent manner (15). Furthermore, pridopidine restores spine density and aberrant calcium signaling (14), all known features of HD. Moreover, pridopidine gene expression profile showed a reversed pattern of the HD disease gene expression profile from a Q175 knock-in (Q175 KI) HD mouse model (15).

WO 2018/039477 discloses a method of maintaining functional capacity, improving functional capacity, or lessening the decline of functional capacity in a human patient including periodically orally administering to the patient a pharmaceutical composition including pridopidine such that a dose of 90-225 mg of pridopidine is administered to the patient per day, so as to thereby maintain functional capacity, improve functional capacity, or lessen the decline of functional capacity in the human patient.

Reilmann et al. discloses a phase 2, randomized, placebo-controlled, multicentre, dose-ranging study on the safety and efficacy of pridopidine in patients with Huntington's disease (PRIDE-HD) (The Lancet Neurology, vol. 18, no. 2, 15 December 2018, pages 165-176).

Coppen et al. discloses current pharmacological approaches to reduce Chorea in Huntington's disease (Drugs, vol. 77, no. 1, 17 December 2016, pages 29-46).

US 2015/374677 discloses analogs of pridopidine having the structure of: 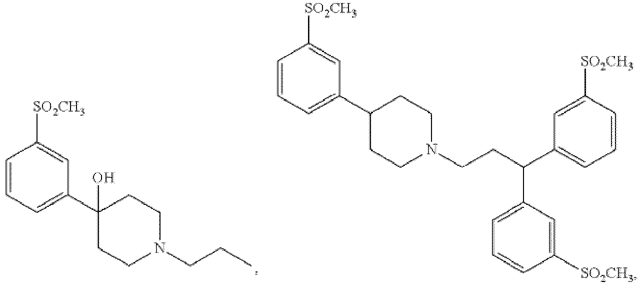 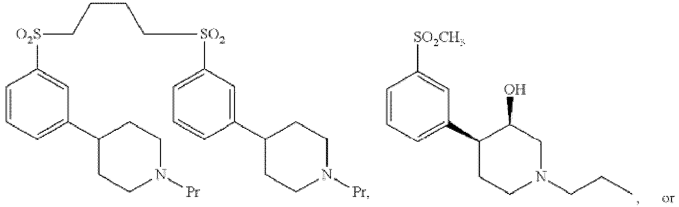 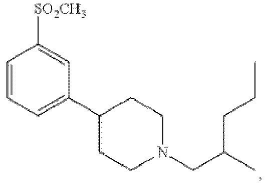 or salt thereof, processes for the preparation thereof, and processes for producing a pridopidine drug product.

### SUMMARY OF THE INVENTION

The present invention provides an oral pharmaceutical composition comprising pridopidine or a pharmaceutically acceptable salt thereof and at least one analog compound of formula 1 or 4 or a pharmaceutically acceptable salt thereof, for use in improving, maintaining or reducing impairment of functional capacity, motor function, cognition, disease progression and quality of life in the treatment of Huntington disease in a human patient afflicted with Huntington disease by orally administering the composition to the patient; wherein the analog compound is represented by the following structures: 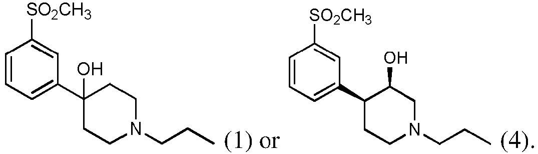 In other embodiments, the composition comprises pridopidine or a pharmaceutically acceptable salt thereof and an analog compound 1 or a pharmaceutically acceptable salt thereof. In other embodiments, the composition comprises pridopidine or a pharmaceutically acceptable salt thereof, and an analog compound 4 or a pharmaceutically acceptable salt thereof. In other embodiments, the composition comprises pridopidine or a pharmaceutically acceptable salt thereof and an analog compound 1 or a pharmaceutically acceptable salt thereof and an analog compound 4 and a pharmaceutically acceptable salt thereof.

In an embodiment, the composition comprises up to 10% w/w of the analog compound or a pharmaceutically acceptable salt thereof.

In an embodiment, the pharmaceutical composition is administered twice per day.

In an embodiment, the human patient's functional capacity is measured by the Unified Huntington's Disease Rating Scale (UHDRS) Total Functional Capacity (TFC).

In an embodiment, the human patient has ≥36 CAG repeats in the Huntingtin gene.

In an embodiment, the composition is administered twice per day and an equal amount of the pharmaceutical composition is administered at each administration.

In an embodiment, the pharmaceutical composition comprising pridopidine or a pharmaceutically acceptable salt thereof is administered at a dose of between 10 mg and 90 mg per day.

In an embodiment, the pharmaceutically acceptable salt is selected from the hydrochloride, hydrobromide, hydroiodide, nitrate, perchlorate, phosphate, acid-phosphate, sulphate, bisulfate, formate, gluconate, glucaronate, saccharate, isonicotinate, acetate, aconate, ascorbate, benzenesulphonate, benzoate, cinnamate, citrate, embonate, enantate, fumarate, glutamate, glycolate, lactate, maleate, gentisinate, malonate, mandelate, methanesulfonate, ethanesulfonate, naphthalene-2-sulphonate, phthalate, salicylate, sorbate, stearate, succinate, tartrate, pantothenate, bitartrate, or toluene-p-sulfonate, pamoate (i.e., 1,1'-methylene-bis-(2-hydroxy-3-naphthoate)) salt.

### BRIEF DESCRIPTION OF THE DRAWINGS

The subject matter regarded as the invention is particularly pointed out and distinctly claimed in the concluding portion of the specification. The invention, however, both as to organization and method of operation, together with objects, features, and advantages thereof, may best be understood by reference to the following detailed description when read with the accompanying drawings in which:

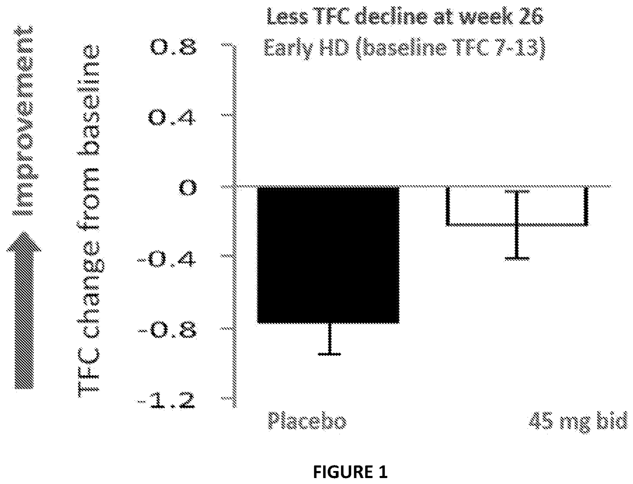

**Figure 1** depicts TFC change from baseline following 26 weeks treatment of early HD patients by administering pridopidine in combination with compound 1.

### DETAILED DESCRIPTION OF THE INVENTION

In the following detailed description, numerous specific details are set forth in order to provide a thorough understanding of the invention. However, it will be understood by those skilled in the art that the present invention may be practiced without these specific details. In other instances, well-known methods, procedures, and components have not been described in detail so as not to obscure the present invention.

The present invention provides an oral pharmaceutical composition comprising pridopidine or a pharmaceutically acceptable salt thereof and at least one analog compound of formula 1 or 4 or a pharmaceutically acceptable salt thereof, for use in improving, maintaining or reducing impairment of functional capacity, motor function, cognition, disease progression and quality of life in the treatment of Huntington disease by orally administering the composition to a human patient; wherein the analog compound is represented by the following structures: 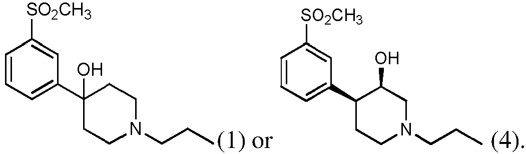 In another embodiment, the pridopidine is pridopidine HCl salt.

In other embodiments, the analog compounds include analog compound 1. In other embodiments, the analog compounds include analog compound 1 and analog compound 4. In other embodiments, the analog compounds include analog compound 4.

In other embodiment, the pharmaceutical composition comprises between 90.0%-99.95% w/w pridopidine or pharmaceutical acceptable salt thereof and between 0.05 % -10% w/w each of the analog compounds or a pharmaceutically acceptable salt thereof. In other embodiment, the pharmaceutical composition comprises up to 10% w/w each of the analog compounds or a pharmaceutically acceptable salt thereof. In other embodiment, the pharmaceutical composition comprises between 0.05% - 1 % w/w w/w each of the analog compounds or a pharmaceutically acceptable salt thereof. In other embodiment, the pharmaceutical composition comprises between 0.5 - 2 % w/w w/w each of the analog compounds or a pharmaceutically acceptable salt thereof. In another embodiment, the pharmaceutical composition comprises between 0.05% - 0.5% w/w each of the analog compounds or a pharmaceutically acceptable salt thereof. In another embodiment, the pharmaceutical composition comprises not more than 0.5% w/w of analog compounds. In another embodiment, the pharmaceutical composition comprises not more than 0.5% w/w of the analog compound 1 or a pharmaceutically acceptable salt thereof. In another embodiment, the pharmaceutical composition comprises not more than 0.5% w/w of the analog compound 4 or a pharmaceutically acceptable salt thereof. In another embodiment, the pharmaceutical composition comprises not more than 0.15% w/w of the analog compound 1 or a pharmaceutically acceptable salt thereof. In another embodiment, the pharmaceutical composition comprises not more than 0.15% w/w of the analog compound 4 or a pharmaceutically acceptable salt thereof.

In one embodiment, the analog compound is analog compound 1 or a pharmaceutically acceptable salt thereof. In another embodiment, the analog compound comprises analog compound 1 and analog compound 4 or a pharmaceutically acceptable salt thereof. In another embodiment, the pharmaceutical composition is administered twice per day. In another embodiment, an equal amount of the pharmaceutical composition is administered at each administration. In another embodiment, the pharmaceutical composition is administered at a daily dose of between 10 mg and 150 mg of pridopidine and at least one analog compound 1 or 4 at a concentration of between 0.05 %- 10% w/w of the composition.
In another embodiment, the pharmaceutical composition is administered at a daily dose of between 10 mg and 150 mg of pridopidine and at least one analog compound 1 or 4 at a concentration not more than 0.5% w/w each of the composition.

In another embodiment, the pharmaceutical composition is administered at a daily dose of between 10 mg and 120 mg of pridopidine and at least one analog compound 1 or 4 at a concentration of between 0.05 %- 10% w/w of the composition. In another embodiment, the pharmaceutical composition is administered at a daily dose of between 10 mg and 100 mg of pridopidine and at least one analog compound 1 or 4 at a concentration of between 0.05 %-10% w/w of the composition. In another embodiment, the pharmaceutical composition is administered at a daily dose of between 10 mg and 90 mg of pridopidine and at least one analog compound 1 or 4 at a concentration of between 0.05 %- 10% w/w of the composition. In another embodiment, the pharmaceutical composition is administered at a daily dose of between 10 mg and 80 mg of pridopidine and at least one analog compound 1 or 4 at a concentration of between 0.05 %- 10% w/w of the composition. In another embodiment, the pharmaceutical composition is administered at a daily dose of between 100 mg and 150 mg of pridopidine and at least one analog compound 1 or 4 at a concentration of between 0.05 %- 10% w/w of the composition. In another embodiment, the pharmaceutical composition is administered at a daily dose of 10 mg, 20 mg, 30 mg, 40 mg,50 mg, 60 mg, 70 mg, 80 mg, 90 mg, 100 mg, 110 mg, 120 mg, 130 mg, 140 mg, 150 mg of pridopidine or any ranges between them, and at least one analog compound 1 or 4 at a concentration of between 0.05 %- 10% w/w of the composition. In another embodiment, the pharmaceutical composition is administered at a dose of 45 mg pridopidine b.i.d (twice a day) and at least one analog compound 1 or 4 at a concentration of between 0.05 %- 10% w/w of the composition.

In an embodiment, the pharmaceutical composition is administered for at least 12 weeks. In another embodiment, the pharmaceutical composition is administered for at least 26 weeks. In another embodiment, the pharmaceutical composition is administered for at least 52 weeks. In another embodiment, the pharmaceutical composition is administered for at least 65 weeks. In another embodiment, the pharmaceutical composition is administered for at least 78 weeks. In another embodiment, the pharmaceutical composition is administered for 1, 2, 3, 4 or 5 years.

One or more symptoms of Huntington's disease may be measured by the Clinician's Interview-based Impression of Change plus Caregiver Input (CIBIC-Plus), Physical Disability Score (PDS), Unified Huntington's Disease Rating Scale (UHDRS) Functional Assessment (FA), Clinical Global Impression of Change (CGI-C), Clinician Global Impression of Severity (CGI-S), Patient Global Impression of Change (PGI-C), Patient Global Impression of Severity (PGI-S), Unified Huntington's Disease Rating Scale (UHDRS) Total Functional Capacity (TFC), Unified Huntington's Disease Rating Scale (UHDRS) Independence Score (IS), HD-Quality of Life scale (HD-QoL), Multiple Sclerosis Walking Scale (MSWS-12), Physical Performance Test (PPT), hand movement score, gait and balance score, Quantitative motor (Q-Motor) assessment, timed up and go (TUG) assessment, cognitive assessment battery (CAB), Symbol Digit Modalities Test (SDMT), Stroop word reading test (SWR), abbreviated Montreal Cognitive Assessment (MoCA) scale, Trail Making Test B assessment, composite Unified Huntington's Disease Ratings Scale (cUHDRS), Huntington Disease Health Index (HD-HI), or Problem Behaviors Assessment-Short form (PBA-s). One or more symptoms may be measured by EQ-5D-5L, Walk-12, or Modified Physical Performance Test (mPPT).

In some embodiments, the motor function impairment is measured by, the composite Unified Huntington's Disease Ratings Scale (cUHDRS), the Huntington Disease Health Index (HD-HI), the Unified Huntington's Disease Rating Scale (UHDRS) Total Motor Score (TMS), the Unified Huntington's Disease Rating Scale (UHDRS) modified Motor Score (mMS), the Unified Huntington's Disease Rating Scale (UHDRS)-Chorea score, the Unified Huntington's Disease Rating Scale (UHDRS)-Dystonia score, Multiple Sclerosis Walking Scale (MSWS-12), Physical Performance Test (PPT), hand movement score, gait and balance score, Quantitative motor (Q-Motor) assessment or by timed up and go (TUG) assessment.

In an embodiment, treating comprises reducing the patient's motor impairment symptoms which are measured by the Unified Huntington Disease Rating Scale (UHDRS) Total Motor Score (TMS).

In an embodiment, treating comprises reducing the patient's motor impairment symptoms which are measured by the Unified Huntington Disease Rating Scale (UHDRS)-Chorea score.

In an embodiment, treating comprises reducing the patient's motor impairment symptoms which are measured by the Unified Huntington Disease Rating Scale (UHDRS)-Dystonia score.

In an embodiment, treating comprises reducing the patient's motor impairment symptoms which are measured by the Unified Huntington Disease Rating Scale (UHDRS) modified Motor Score (mMS).

In an embodiment, the patient is at least 21 years old. In another embodiment, the patient is less than 30 years old.

In some embodiments, the HD patient is an adult patient. In some embodiments, the HD patient is an early stage HD patient (TFC 7-13). In some embodiments, the patient is a stage 1 HD (HD1, TFC 11-13) patient or stage 2 HD (HD2, TFC 7-10) patient. In some embodiments, the patient is HD1 patient and is experiencing one or more symptom of HD. In some embodiments, the HD patient is not a pre-manifest HD patient. In some embodiments, the patient has a baseline TFC score of 7-13 or at least 7, at least 8, at least 9, at least 10, at least 11, at least 12, 13 or 7-10 or 11-13.

In an embodiment, the patient has a UHDRS-TMS score ≥20 before beginning treatment.

In an embodiment, the patient has a UHDRS-IS below or equal to 90% before beginning treatment.

In an embodiment, the patient has ≥36 CAG repeats in the Huntingtin gene. In some embodiments, the HD patient has been diagnosed as having at least 36 CAG repeats in the huntingtin gene.

In an embodiment, the pridopidine or a pharmaceutically acceptable salt thereof is pridopidine hydrochloride. In another embodiment, the pridopidine or a pharmaceutically acceptable salt thereof is pridopidine hydrobromide.

In other embodiments, the pharmaceutically acceptable salt is selected from hydrochloride, hydrobromide, hydroiodide, nitrate, perchlorate, phosphate, acid-phosphate, sulphate, bisulfate, formate, gluconate, glucaronate, saccharate, isonicotinate, acetate, aconate, ascorbate, benzenesulphonate, benzoate, cinnamate, citrate, embonate, enantate, fumarate, glutamate, glycolate, lactate, maleate, gentisinate, malonate, mandelate, methanesulfonate, ethanesulfonate, naphthalene-2-sulphonate, phthalate, salicylate, sorbate, stearate, succinate, tartrate, pantothenate, bitartrate, or toluene-p-sulfonate, pamoate (i.e., 1,1'-methylene-bis-(2-hydroxy-3-naphthoate)) salt. In another embodiment, the salt is HCl salt.

In some embodiments, the pharmaceutical composition comprises one or more pharmaceutically acceptable carriers or excipients.

In an embodiment, the pharmaceutically acceptable carriers or excipients are selected from: binder, filler, plasticizer, glidant and lubricant or mixtures thereof.

In an embodiment, the binder is selected from: starch, pregeletinized starch, polyethylene oxide, cellulose polymers, hydroxypropylmethyl cellulose, hydroxypropylcellulose, methylcellulose, hydroxyethyl cellulose, polyvinylpyrrolidone, polyvinyl alcohol or mixtures thereof.

In an embodiment, the filler is selected from: microcrystalline cellulose, sugar spheres, lactose, sorbitol, dextrose, sucrose, mannitol, dibasic or tribasic calcium phosphate, calcium sulfate, starch, retalac or mixtures thereof.

In an embodiment, the filler is microcrystalline cellulose and is a silicified microcrystalline cellulose.

In an embodiment, the filler is lactose. In another embodiment, the filler is a mixture of microcrystalline cellulose and lactose, and wherein the microcrystalline cellulose and is a silicified microcrystalline cellulose. In an embodiment, the filler is microcrystalline cellulose and is a silicified microcrystalline cellulose.

In an embodiment, the pharmaceutical composition comprises silicified microcrystalline cellulose and magnesium stearate as excipients.

In an embodiment, the lubricant is selected from: sodium stearyl fumarate, stearic acid, magnesium stearate, calcium stearate, zinc stearate, talc, glyceryl behenate, glyceryl monostearate, or mixtures thereof.

In an embodiment, the lubricant is magnesium stearate.

In an embodiment, the glidant is selected from: starch, pregelatinized starch, silicone dioxide, colloidal silicone dioxide, talc or mixtures thereof.

In an embodiment, the glidant is colloidal silicone dioxide.

In an embodiment, treating comprises reducing the patient's motor impairment symptoms which are measured by the Unified Huntington's Disease Rating Scale (UHDRS) Total Motor Score (TMS).

In an embodiment, treating comprises reducing the patient's motor impairment symptoms which are measured by the Unified Huntington's Disease Rating Scale (UHDRS) modified Motor Score (mMS).

In an embodiment, treating comprises reducing the patient's motor impairment symptoms which are measured by the Unified Huntington's Disease Rating Scale (UHDRS)-Chorea score.

In an embodiment, treating comprises reducing the patient's motor impairment symptoms which are measured by the Unified Huntington's Disease Rating Scale (UHDRS)-Dystonia score.

The active compounds for use according to the invention may be provided in any form suitable for the intended administration. Suitable forms include pharmaceutically (i.e. physiologically) acceptable salts of the compound of the invention.

### Pharmaceutical Compositions

While the compounds for use according to the invention may be administered in the form of the raw compound, it is preferred to introduce the active ingredients, optionally in the form of physiologically acceptable salts, in a pharmaceutical composition together with one or more adjuvants, excipients, carriers, buffers, diluents, and/or other customary pharmaceutical auxiliaries.

In an embodiment, the pharmaceutical compositions comprise the active compound, pharmaceutically acceptable salt or derivative thereof, together with one or more pharmaceutically acceptable carriers therefore, and, optionally, other therapeutic and/or prophylactic ingredients know and used in the art. The carrier(s) must be "acceptable" in the sense of being compatible with the other ingredients of the formulation and not harmful to the recipient thereof.

Oral administration may include in particular in tablet, in capsule, in beads, minitablets, multiparticulates, in powder, or in liquid form, and parenteral administration, in particular cutaneous, ophthalmic, eye drops, subcutaneous, intramuscular, or intravenous injection. The pharmaceutical composition of the invention can be manufactured by the skilled person by use of standard methods and conventional techniques appropriate to the desired formulation. When desired, compositions adapted to give sustained release of the active ingredient may be employed.

In some embodiments, multiparticulates or multiple unit dosage forms are the discrete, small, repetitive units of drug particles which may or may not possess similar drug release pattern.

Further details on techniques for formulation and administration may be found in the latest edition of Remington's Pharmaceutical Sciences (Maack Publishing Co., Easton, PA).

As used herein, "effective" as in an amount effective to achieve an end means the quantity of a component that is sufficient to yield an indicated therapeutic response without undue adverse side effects (such as toxicity, irritation, or allergic response) commensurate with a reasonable benefit/risk ratio when used in the manner of this disclosure. For example, an amount effective to treat a movement disorder. The specific effective amount varies with such factors as the particular condition being treated, the physical condition of the patient, the type of mammal being treated, the duration of the treatment, the nature of concurrent therapy (if any), and the specific formulations employed and the structure of the compounds or its derivatives.

As used herein, an amount of pridopidine as measured in milligrams refers to the milligrams of pridopidine (4-[3-(methylsulfonyl)phenyl]-1-propyl-piperidine) present in a preparation, regardless of the form of the preparation. For example, a unit dose containing "90 mg pridopidine" means the amount of pridopidine base in a preparation is 90 mg, regardless of the form of the preparation. Thus, when in the form of a salt, e.g. pridopidine hydrochloride, the weight of the salt form necessary to provide a dose of 90 mg pridopidine would be greater than 90 mg due to the presence of the salt.

As used herein, to "treat" or "treating" encompasses, e.g., reducing a symptom, inducing inhibition, regression, or stasis of the disorder and/or disease. As used herein, "inhibition" of disease progression or disease complication in a subject means preventing or reducing the disease progression and/or disease complication in the subject.

### Listing of Abbreviations and Definitions of Terms

The following abbreviations are used throughout this application:
AE: adverse event; ALT: alanine aminotransferase; AR: Autoregressive; Arc mRNA: activity-regulated cytoskeleton-associated protein messenger ribonucleic acid; ARH: Heterogeneous Autoregressive; AST: aspartate aminotransferase; AUC: area under the concentration-time curve; Bid: twice daily; BL = Baseline; CAB: cognitive assessment battery; CAG: cytosine-adenosine-guanine; CDMS: clinical data management system; CFR: Code of Federal Regulations; CGI-C: Clinical Global Impression of Change; CGI-S: Clinical Global Impression of Severity; CI: confidence interval; CIBIC-Plus: Clinician's Interview-based Impression of Change plus Caregiver Input; CIBIS: Clinician's Interview-based Impression of Severity; CIOMS: Council for International Organizations of Medical Sciences; Cmax: maximum observed plasma drug concentration; CNS: central nervous system; CRF: case report form; CRO: contract research organization; CS: Compound Symmetry; CSH: Heterogeneous Compound Symmetry; C-SSRS: Columbia-Suicide Severity Rating Scale; CYP: cytochrome P450; DSM-IV TR: Diagnostic and Statistical Manual - Fourth Edition Text Revision; ECG: electrocardiogram; EM: extensive metabolizers; EU: European Union; FA: Functional Assessment; FAS: full analysis set; FDA: US Food and Drug Administration; Freq: tapping frequency; GCP: Good Clinical Practice; GFV-C: grip force variability in the static phase; GGT: gamma-glutamyl transpeptidase; HART: Huntington's disease ACR16 Randomized Trial; HCG: human chorionic gonadotropin; HD: Huntington disease; HD-QoL = Huntington's disease Quality of Life; ICH: International Conference on Harmonisation; IEC: Independent Ethics Committee; IOI: inter onset interval; IPI: inter peak interval; IRB: Institutional Review Board; IRT: interactive response technology; IS: Independence Score; ITI: inter tap interval; ITT: intent-to-treat; LSO: local safety officer; MAD: multiple ascending dose; MedDRA: Medical Dictionary for Regulatory Activities; MermaiHD: Multinational European Multicentre ACR16 study in Huntington's Disease; ML: Maximum-Likelihood; mMS: Modified Motor Score; MoCA: Montreal cognitive assessment; MS: Multiple sclerosis; MSWS-12: Multiple Sclerosis Walking Scale; MTD: maximum tolerated dose; NMDA: N-methyl-D-aspartate; NOAEL: no observed adverse effect level; PBA-s: Problem Behaviors Assessment-Short form; PD: pharmacodynamic(s); PDS: Physical disability scale; PK: pharmacokinetic(s); PM: poor metabolizer; PPT: physical performance test; Qd: once daily; Q-Motor: Quantitative motor; QoL: Quality of life; QTcF: Fridericia-corrected QT interval; RBC: red blood cell; REML: Restricted Maximum-Likelihood; SAE: serious adverse event; SD: standard deviation; SDMT: symbol digit modalities test; SOC: system organ class; SOP: standard operating procedure; SUSAR: suspected unexpected serious adverse reaction; t½: half life; TC = telephone call; TD: tap duration; TF: tapping force; TFC: Total functional capacity; TMS: Total Motor Score; TUG: timed up an go; UHDRS: Unified Huntington's Disease Rating Scale; ULN: upper limit of the normal range; US: United States; WBC: white blood cell; WHO: World Health Organization; WHO: Drug World Health Organization (WHO) drug dictionary; ΔHR: change from baseline in heart rate; ΔQTcF: change from baseline in QTcF; ΔΔHR: placebo-corrected change from baseline in heart rate; Placebo-Controlled Study-Huntington's Disease; ΔΔQTcF: placebo-corrected change from baseline in QTcF

### EXAMPLES

### Example 1:

### Analog compounds are selective to S1R

A binding affinity study was conducted using several analog compounds of this invention. Compounds were tested at multiple concentrations between 1.0E-09 M to 1.0E-04 M. The binding of each compound analog to the S1R and to the S2R was calculated as the percent of inhibition of the binding of a radioactively labeled ligand specific for each target. It was found that all analogs are S1R ligands and are also highly selective for S1R showing low or no binding to the S2R.

**Table 1:**

| | S1R Ki (µM) | S2R Ki(µM) | S1R fold selectivity(S2R/S1R) |
|---|---|---|---|
| Pridopidine* | 0.057 | 5.45 | 96 |
| Analog compound 6 | 0.007 | 0.18 | 26 |
| Analog compound 1 | 0.37 | >100 | >270 |
| Analog compound 2 | 1.8 | >100 | >56 |
| Analog compound 4 | 2.9 | >100 | >35 |

| | | | |
|---|---|---|---|
| *From Johnson et al. 2019. | | | |

These results support the use of the combination of pridopidine and analog compounds 1-7 for use in treating HD and/or improving, maintaining or reducing impairment of functional capacity of a human patient afflicted with Huntington disease and/or improving, maintaining or reducing motor function impairment of a human patient afflicted with Huntington disease.

### Example 2:

### Functional capacity using the combination of Pridopidine and analog compounds of this invention

Figure 1 presents total functional capacity (TFC) results of early stage HD patients after 26 weeks treatment of 45 mg b.i.d (twice a day) pridopidine and analog compound 1.

Table 2 presents the results of TFC using pridopidine + analog compound 1. As can be seen by using the combination, less TFC decline at week 26.

**Table 2: TFC change from baseline at week 26 of early stage HD patients using 45 mg b.i.d pridopidine in combination with analog 1.**

| | **Placebo** | **45 mg pridopidine plus compound 1 b.i.d** |
|---|---|---|
| **n** | 62 | 59 |
| **Δ pridopidine vs placebo** | | 0.56 |
| **p value** | | 0.0359 |

| | | |
|---|---|---|
| \ | | |

### Example 3

### Sub Domains of Functional Capacity using the combination of Pridopidine and analog compounds of this invention

**Table 3: Change from baseline to week 26 in early HD patients (baseline TFC 7-13) of the TFC subdomains: Finances, Activity of daily living (ADL), Domestic chores, Care level and Occupation.**

| Early HD population (baseline TFC 7-13) | Placebo (N=62) | 45 mg bid plus compound 1 (N=59) |
|---|---|---|
| **Finances** | | |
| mean (SE*) change from baseline | -0.24 (0.08) | -0.04 (0.09) |
| mean (95%CI) difference vs. placebo | | 0.2 (-0.03, 0.44) |
| Nominal P value vs. placebo | | 0.085 |

| **ADL** | | |
|---|---|---|
| mean (SE*) change from baseline | -0.27 (0.07) | -0.03 (0.07) |
| mean (95%CI) difference vs. placebo | | 0.24 (0.04, 0.44) |
| Nominal P value vs. placebo | | 0.012 |

| **Care Level** | | |
|---|---|---|
| mean (SE*) change from baseline | -0.04 (0.02) | +0.01 (0.02) |

| **Occupation** | | |
|---|---|---|
| mean (SE*) change from baseline | -0.12 (0.07) | -0.08 (0.07) |

| **Domestic Chores** | | |
|---|---|---|
| mean (SE*) change from baseline | -0.13 (0.06) | -0.08 (0.06) |

| | | |
|---|---|---|
| *SE refers to standard error. | | |

Table 3 presents the results of the five sub-domains composing the total functional capacity (TFC) in early stage HD patients after 26 weeks treatment with placebo and 45 mg b.i.d (twice a day) pridopidine plus compound 1. Negative change indicates worsening. In all 5 subdomains placebo showed a stronger decline compared to patients treated with pridopidine + compound 1.

ADL placebo change from baseline is -0.27 points vs pridopidine + compound 1showing minimal decline of -0.03 points (difference pridopidine + compound 1 vs placebo is 0.24, p=0.012). In Finances, placebo deteriorated by -0.24 points from baseline, vs minimal change in pridopidine + compound 1 of -0.04 (difference pridopidine + compound 1 vs placebo is 0.2, p=0.085). In Care level, placebo group deteriorated by -0.04 points compared to the observed maintenance in the pridopidine + compound 1 group of +0.01 points. Domestic chores: placebo declines from baseline in --0.13 points compared to minimal change in the pridopidine + compound 1 group of -0.08 points change from baseline. Similarly, in occupation, placebo group showed -0.12 decline from baseline, vs a minimal change in the pridopidine + compound 1 group of -0.08 points.

### REFERENCES CITED:

1. Mahant N, McCusker EA, Byth K, Graham S. Huntington's disease: Clinical correlates of disability and progression. Neurology. 2003.
2. Tabrizi SJ, Reilmann R, Roos RAC, Durr A, Leavitt B, Owen G, et al. Potential endpoints for clinical trials in premanifest and early Huntington's disease in the TRACK-HD study: Analysis of 24 month observational data. Lancet Neurol. 2012.
3. Tabrizi SJ, Scahill RI, Owen G, Durr A, Leavitt BR, Roos RA, et al. Predictors of phenotypic progression and disease onset in premanifest and early-stage Huntington's disease in the TRACK-HD study: Analysis of 36-month observational data. Lancet Neurol. 2013.
4. Mestre T, Ferreira J, Coelho MM, Rosa M, Sampaio C. Therapeutic interventions for disease progression in Huntington's disease. Cochrane Database of Systematic Reviews. 2009.
5. Mestre T, Ferreira J, Coelho MM, Rosa M, Sampaio C. Therapeutic interventions for symptomatic treatment in Huntington's disease. Cochrane Database of Systematic Reviews. 2009.
6. Frank S, Testa CM, Stamler D, Kayson E, Davis C, Edmondson MC, et al. Effect of deutetrabenazine on chorea among patients with huntington disease: A randomized clinical trial. JAMA - J Am Med Assoc. 2016.
7. Shen V, Clarence-Smith K, Hunter C, Jankovic J. Safety and Efficacy of Tetrabenazine and Use of Concomitant Medications During Long-Term, Open-Label Treatment of Chorea Associated with Huntington's and Other Diseases. Tremor Other Hyperkinet Mov (N Y). 2013.
8. Johnston TH, Geva M, Steiner L, Orbach A, Papapetropoulos S, Savola J-M, et al. Pridopidine, a clinic-ready compound, reduces 3,4-dihydroxyphenylalanine-induced dyskinesia in Parkinsonian macaques. Mov Disord [Internet]. 2018 Dec 21 [cited 2019 Mar 11]; Available from: http://doi.wiley.com/10.1002/mds.27565.
9. Hayashi T, Su T-. The Sigma Receptor: Evolution of the Concept in Neuropsychopharmacology. Curr Neuropharmacol. 2005;3(4):267-80.
10. Su TP, Hayashi T, Maurice T, Buch S, Ruoho AE. The sigma-1 receptor chaperone as an inter-organelle signaling modulator. Trends in Pharmacological Sciences. 2010.
11. Eddings CR, Arbez N, Akimov S, Geva M, Hayden MR, Ross CA. Pridopidine protects neurons from mutant-huntingtin toxicity via the sigma-1 receptor. Neurobiol Dis. 2019.
12. Ionescu A, Gradus T, Altman T, Maimon R, Saraf Avraham N, Geva M, et al. Targeting the Sigma-1 Receptor via Pridopidine Ameliorates Central Features of ALS Pathology in a SOD1G93A Model. Cell Death Dis [Internet]. 2019;10(3):210. Available from: http://www.nature.com/articles/s41419-019-1451-2.
13. Smith-Dijak AI, Nassrallah WB, Zhang LYJ, Geva M, Hayden MR, Raymond LA. Impairment and restoration of homeostatic plasticity in cultured cortical neurons from a mouse model of huntington disease. Front Cell Neurosci. 2019.
14. Ryskamp D, Wu J, Geva M, Kusko R, Grossman I, Hayden M, et al. The sigma-1 receptor mediates the beneficial effects of pridopidine in a mouse model of Huntington disease. Neurobiol Dis [Internet]. 2017 Jan [cited 2019 Mar 12];97(Pt A):46-59. Available from: http://www.ncbi.nlm.nih.gov/pubmed/27818324.
15. Geva M, Kusko R, Soares H, Fowler KD, Birnberg T, Barash S, et al. Pridopidine activates neuroprotective pathways impaired in Huntington Disease. Hum Mol Genet [Internet]. 2016 [cited 2019 Mar 12];25(18):3975-87. Available from: http://www.ncbi.nlm.nih.gov/pubmed/27466197.

## Claims

1. An oral pharmaceutical composition comprising pridopidine or a pharmaceutically acceptable salt thereof and at least one analog compound of formula 1 or 4 or a pharmaceutically acceptable salt thereof, for use in improving, maintaining or reducing impairment of functional capacity, motor function, cognition, disease progression and quality of life in the treatment of Huntington disease by orally administering the composition to a human patient; wherein the analog compound is represented by the following structures:

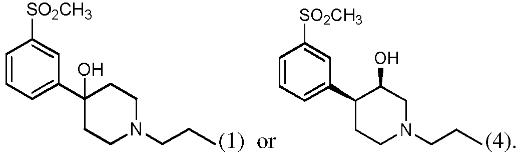

2. The composition for use according to claim 1, wherein the composition comprises pridopidine or a pharmaceutically acceptable salt thereof and analog compound 1 or a pharmaceutically acceptable salt thereof; or
the composition comprises pridopidine or a pharmaceutically acceptable salt thereof, and analog compound 4 or a pharmaceutically acceptable salt thereof; or
the composition comprises pridopidine or a pharmaceutically acceptable salt thereof, analog compound 1 or a pharmaceutically acceptable salt thereof and analog compound 4 or a pharmaceutically acceptable salt thereof

3. The composition for use according to claim 1, wherein the composition comprises up to 10% w/w of the analog compound or a pharmaceutically acceptable salt thereof.

4. The composition for use according to claim 1, wherein the pharmaceutical composition is administered twice per day.

5. The composition for use according to claim 1, wherein human patient's functional capacity is measured by the Unified Huntington's Disease Rating Scale (UHDRS) Total Functional Capacity (TFC).

6. The composition for use according to any of claims 1-5, wherein the human patient has ≥36 CAG repeats in the Huntingtin gene.

7. The composition for use according to any of claims 1-6, wherein the composition is administered twice per day and an equal amount of the pharmaceutical composition is administered at each administration.

8. The composition for use according to any of claims 1-7, wherein the pharmaceutical composition comprising pridopidine or a pharmaceutically acceptable salt thereof is administered at a dose of between 10 mg and 90 mg per day.

9. The composition for use according to any of claims 1-8, wherein the pharmaceutically acceptable salt is selected from the hydrochloride, hydrobromide, hydroiodide, nitrate, perchlorate, phosphate, acid-phosphate, sulphate, bisulfate, formate, gluconate, glucaronate, saccharate, isonicotinate, acetate, aconate, ascorbate, benzenesulphonate, benzoate, cinnamate, citrate, embonate, enantate, fumarate, glutamate, glycolate, lactate, maleate, gentisinate, malonate, mandelate, methanesulfonate, ethanesulfonate, naphthalene-2-sulphonate, phthalate, salicylate, sorbate, stearate, succinate, tartrate, pantothenate, bitartrate, and toluene-p-sulfonate, pamoate (i.e., 1,1'-methylene-bis-(2-hydroxy-3-naphthoate)) salt.

## Patentansprüche

1. Orale pharmazeutische Zusammensetzung, enthaltend Pridopidin oder ein pharmazeutisch verträgliches Salz davon und mindestens eine analoge Verbindung der Formel 1 oder 4 oder ein pharmazeutisch verträgliches Salz davon, zur Verwendung beim Verbessern, Erhalten oder Verringern der Beeinträchtigung der funktionalen Fähigkeit, der motorischen Funktion, der Kognition, des Fortschreitens der Krankheit und der Lebensqualität bei der Behandlung von Morbus Huntington durch orale Verabreichung der Zusammensetzung an einen menschlichen Patienten; wobei die analoge Verbindung durch die folgenden Strukturen dargestellt wird:

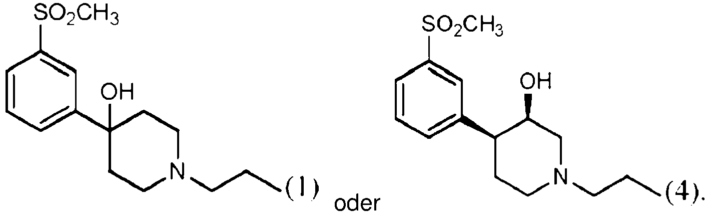

2. Zusammensetzung zur Verwendung nach Anspruch 1, wobei die Zusammensetzung Pridopidin oder ein pharmazeutisch verträgliches Salz davon und die analoge Verbindung 1 oder ein pharmazeutisch verträgliches Salz davon enthält; oder
die Zusammensetzung Pridopidin oder ein pharmazeutisch verträgliches Salz davon und die analoge Verbindung 4 oder ein pharmazeutisch verträgliches Salz davon enthält; oder
die Zusammensetzung Pridopidin oder ein pharmazeutisch verträgliches Salz davon, die analoge Verbindung 1 oder ein pharmazeutisch verträgliches Salz davon und die analoge Verbindung 4 oder ein pharmazeutisch verträgliches Salz davon enthält.

3. Zusammensetzung zur Verwendung nach Anspruch 1, wobei die Zusammensetzung bis zu 10 Gew.-% der analogen Verbindung oder eines pharmazeutisch verträglichen Salzes davon enthält.

4. Zusammensetzung zur Verwendung nach Anspruch 1, wobei die pharmazeutische Zusammensetzung zweimal täglich verabreicht wird.

5. Zusammensetzung zur Verwendung nach Anspruch 1, wobei die funktionale Fähigkeit des menschlichen Patienten anhand der Total Functional Capacity (TFC) der Unified Huntington's Disease Rating Scale (UHDRS) gemessen wird.

6. Zusammensetzung zur Verwendung nach einem der Ansprüche 1-5, wobei der menschliche Patient ≥36 CAG-Wiederholungen in dem Huntingtin-Gen aufweist.

7. Zusammensetzung zur Verwendung nach einem der Ansprüche 1-6, wobei die Zusammensetzung zweimal täglich verabreicht wird und bei jeder Verabreichung die gleiche Menge der pharmazeutischen Zusammensetzung verabreicht wird.

8. Zusammensetzung zur Verwendung nach einem der Ansprüche 1-7, wobei die pharmazeutische Zusammensetzung, die Pridopidin oder ein pharmazeutisch verträgliches Salz davon enthält, in einer Dosis zwischen 10 mg und 90 mg täglich verabreicht wird.

9. Zusammensetzung zur Verwendung nach einem der Ansprüche 1-8, wobei das pharmazeutisch verträgliche Salz ausgewählt ist aus dem Hydrochlorid-, Hydrobromid-, Hydroiodid-, Nitrat-, Perchlorat-, Phosphat-, Säurephosphat-, Sulfat-, Bisulfat-, Formiat-, Gluconat-, Glucaronat-, Saccharat-, Isonicotinat-, Acetat-, Aconat-, Ascorbat-, Benzolsulfonat-, Benzoat-, Cinnamat-, Citrat-, Embonat-, Enantat-, Fumarat-, Glutamat-, Glycolat-, Lactat-, Maleat-, Gentisinat-, Malonat-, Mandelat-, Methansulfonat-, Ethansulfonat-, Naphthalin-2-sulfonat-, Phthalat-, Salicylat-, Sorbat-, Stearat-, Succinat-, Tartrat-, Pantothenat-, Bitartrat- und Toluol-p-sulfonat-, Pamoat (d. h. 1,1'-Methylen-bis-(2-hydroxy-3-naphthoat))-Salz.

## Revendications

1. Composition pharmaceutique orale comprenant de la pridopidine ou un sel pharmaceutiquement acceptable de celle-ci et au moins un composé analogue de formule 1 ou 4 ou un sel pharmaceutiquement acceptable de celui-ci, destinée à être utilisée pour améliorer, maintenir ou réduire l'altération de la capacité fonctionnelle, de la fonction motrice, de la cognition, de la progression de la maladie et de la qualité de vie dans le traitement de la maladie de Huntington par l'administration par voie orale de la composition à un patient humain ; le composé analogique étant représenté par les structures suivantes :

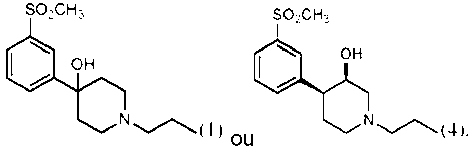

2. Composition selon la revendication 1, dans laquelle la composition comprend de la pridopidine ou un sel pharmaceutiquement acceptable de celle-ci et un composé analogue 1 ou un sel pharmaceutiquement acceptable de celui-ci ; ou
la composition comprend de la pridopidine ou un sel pharmaceutiquement acceptable de celle-ci, et un composé analogue 4 ou un sel pharmaceutiquement acceptable de celui-ci ; ou
la composition comprend de la pridopidine ou un sel pharmaceutiquement acceptable de celle-ci, un composé analogue 1 ou un sel pharmaceutiquement acceptable de celui-ci et un composé analogue 4 ou un sel pharmaceutiquement acceptable de celui-ci.

3. Composition selon la revendication 1, dans laquelle la composition comprend jusqu'à 10 % p/p du composé analogue ou d'un sel pharmaceutiquement acceptable de celui-ci.

4. Composition selon la revendication 1, dans laquelle la composition pharmaceutique est administrée deux fois par jour.

5. Composition selon la revendication 1, dans laquelle la capacité fonctionnelle d'un patient humain est mesurée par la capacité fonctionnelle totale (TFC) selon l'échelle d'évaluation unifiée de la maladie de Huntington (UHDRS).

6. Composition selon l'une quelconque des revendications 1 à 5, dans laquelle le patient humain présente plus de 36 répétitions CAG dans le gène de la Huntingtine.

7. Composition selon l'une quelconque des revendications 1 à 6, dans laquelle la composition est administrée deux fois par jour et une quantité égale de la composition pharmaceutique est administrée à chaque administration.

8. Composition selon l'une quelconque des revendications 1 à 7, dans laquelle la composition pharmaceutique comprenant de la pridopidine ou un sel pharmaceutiquement acceptable de celle-ci est administrée à une dose comprise entre 10 et 90 mg par jour.

9. Composition selon l'une quelconque des revendications 1 à 8, dans laquelle le sel pharmaceutiquement acceptable est choisi parmi le chlorhydrate, le bromhydrate, l'hydriodure, le nitrate, le perchlorate, le phosphate, le phosphate acide, le sulfate, le bisulfate, le formiate, le gluconate, le glucaronate, le saccharate, l'isonicotinate, l'acétate, l'aconate, l'ascorbate, le benzènesulfonate, le benzoate, le cinnamate, le citrate, l'embonate, l'énantate, le fumarate, le glutamate, le glycolate, le lactate, le maléate, le gentisinate, le malonate, le mandélate, le méthanesulfonate, l'éthanesulfonate, le naphtalène-2-sulfonate, le phtalate, le salicylate, le sorbate, le stéarate, le succinate, le tartrate, le pantothénate, le bitartrate et le toluène-p-sulfonate, ainsi que le sel de pamoate (c.-à-d. 1,1'-méthylène-bis-(2-hydroxy-3-naphtoate).
